# EUROPÄISCHE PATENTSCHRIFT

(11) **EP 2 333 092 B1**
(45) Veröffentlichungstag und Bekanntmachung des Hinweises auf die Patenterteilung: **03.10.2012**
(21) Anmeldenummer: 09178390.2
(22) Anmeldetag: 08.12.2009
(51) Int. Cl.: C12P 7/10, C12P 7/06, C07C 29/76

(54) **Verfahren zur Gewinnung von Ethanol während einer Fermentation**
Method for the recovery of ethanol during fermentation
Procédé de recupération d'éthanol pendant une fermentation

(43) Veröffentlichungstag der Anmeldung: 15.06.2011
(73) Patentinhaber: Süd-Chemie IP GmbH & Co. KG, 80333 München (DE)
(72) Erfinder: Zavrel, Michael, 81379 München (DE); Kraus, Michael, 82178 Puchheim (DE); Hofmann, Sandra, 97285 Röttingen (DE); Koltermann, Andre, 82057 Icking (DE); Ott, Christian, 84539 Ampfing (DE); Dragovic, Zdravko, 81375 München (DE)
(74) Vertreter: HOFFMANN EITLE

(56) Entgegenhaltungen:
- US-A- 4 515 892
- US-A- 4 665 027
- DATABASE ENERGY CITATIONS DATABASE 8. Februar 1983 (1983-02-08), Walsh P K et al: "Ethanol separation from water in a 2-stage adsorption process" XP002581550 Database accession no. 6838688 & BIOTECHNOLOGY AND BIOENGINEERING SYMPOSIUM, NO. 13. 5TH SYMPOSIUM ON BIOTECHNOLOGY FOR FUELS AND CHEMICALS; GATLINBURG, TENN., USA, P. JOHN WILEY AND SONS, INC., NEW YORK, N.Y., USA., 10. Mai 1983 (1983-05-10), Seiten 629-647,
- VANE L M: "Separation technologies for the recovery and dehydration of alcohols from fermentation broths" BIOFUELS, BIOPRODUCTS AND BIOREFINING 2008 NOVEMBER/DECEMBER JOHN WILEY AND SONS LTD GBR LNKD- DOI:10.1002/BBB.108, Bd. 2, Nr. 6, November 2008 (2008-11), Seiten 553-588, XP002581551
- DOMINGUEZ JOSE M ET AL: "Ethanol production from xylose with the yeast Pichia stipitis and simultaneous product recovery by gas stripping using a gas-lift loop fermentor with attached side-arm (GLSA)" BIOTECHNOLOGY AND BIOENGINEERING, Bd. 67, Nr. 3, 5. Februar 2000 (2000-02-05), Seiten 336-343, XP002581552 ISSN: 0006-3592
- DATABASE WPI Week 200830 Thomson Scientific, London, GB; AN 2008-E26493 XP002581553 & CN 101 024 846 A (UNIV FUDAN) 29. August 2007 (2007-08-29)
- DATABASE EPODOC [Online] EUROPEAN PATENT OFFICE, THE HAGUE, NL; 17. September 2008 (2008-09-17), XP002581554 Database accession no. CN-200810036646-A & CN 101 265 159 A (SHANGHAI LEAD BIOTECHNOLOGY CO [CN]) 17. September 2008 (2008-09-17)
- DATABASE WPI Week 200873 Thomson Scientific, London, GB; AN 2008-M35536 XP002581555 & CN 101 225 017 A (UNIV FUDAN) 23. Juli 2008 (2008-07-23)
- Yang, R. T.: "Gas separation by adsorption processes" 1997, Imperial College Press , London , XP002581721 , Seiten 1-8, 23-26 * Seite 1 - Seite 8; Tabelle 2.3 * * Seite 23 - Seite 26 *

## Beschreibung

### Gebiet der Erfindung

Die Erfindung betrifft ein Verfahren zur Herstellung von Ethanol durch Fermentation.

### Hintergrund der Erfindung

Bei der Fermentation kohlenhydrathaltiger Rohsubstrate können Hefen oder Bakterien die Zuckermonomere bestehend aus fünf ("C5-Zucker", Pentosen) und / oder aus sechs Kohlenstoffatomen ("C6-Zucker", Hexosen) zu Ethanol umsetzen (Huber et al., Chem. Rev., 2006, Vol. 106, S. 4044-4098). Ethanol wird als "Bioethanol" bezeichnet, sofern es aus biogenen Rohstoffen hergestellt wird. Bioethanol eignet sich als Biokraftstoff, als Beimischung zu Ottokraftstoffen oder zur chemischen Weiterverarbeitung und wird bisher hauptsächlich aus Zucker sowie stärkehaltigem Getreide gewonnen, bislang jedoch nicht in nennenswerten Mengen aus lignocellulosischer Biomasse (LCB) (Huber et al., Chem. Rev., 2006, Vol. 106, S. 4044-4098; Kamm und Kamm, Chem. Ing. Tech., 2007, Vol. 79, S. 592-603).

Für die Herstellung von Ethanol durch Fermentation ist eine Trennung des Ethanols von der Fermentationslösung erforderlich. Zu diesen Techniken zählen Pervaporation, Extraktion, Adsorption, Umkehrosmose und Gas-Stripping (Windsperger et al., Verfahrenstechnik, 1989, Vol. 23, S. 16-21; Qureshi et al., Bioprocess Biosyst. Eng., 2005, Vol. 27, S. 215-222).. Gas-Stripping ist eine selektive Entfernung volatiler Substanzen aus der Fermentationslösung (Ezeji et al., J. lnd. Microbiol. Biotechnol., 2007, Vol. 34, 771-777).

Das Ethanol muss nach dem Strippen von dem Ethanol-Trägergas-Gemisch abgetrennt werden. Hierfür stehen unterschiedliche Techniken zur Verfügung, wie z.B. die Kondensation oder selektive Adsorption des Ethanols an einen Adsorber. So beschreibt CA 1 195 258 ein Verfahren, bei dem nach erfolgter Fermentierung die Fermentationslösung einem Gas-Stripping unterworfen und das Ethanol-Trägergas-Gemisch dann an einem Molekularsieb unter Bedingungen adsorbiert wird, bei denen die Kapillarkondensation von Wasser vermieden wird. Dieses Verfahren erlaubt aber nicht, die Ethanolkonzentration während der Fermentation zu kontrollieren.

Eine solche Kontrolle der Ethanolkonzentration in der Fermentationslösung ist jedoch für eine industrielle Bioethanolherstellung wichtig. Ein Problem bei der Produktion von Bioethanol stellt die zunehmende inhibierende Wirkung und der toxische Einfluss des gebildeten Ethanols auf die Mikroorganismen während der Fermentation dar. Aufgrund der inhibierenden Wirkung und des toxischen Einflusses von während der Fermentation gebildeten Produkten wurden diverse Techniken entwickelt, um diese in-*situ* während der Fermentation abzutrennen.

So beschreiben Walsh et al. (Biotechnology and Bioengineering Symp., Nr. 13, 1983, S. 629 ― 647) ein Verfahren, bei dem C6-Zucker zu Ethanol fermentiert und das Ethanol *in situ* aus dem Fermenter durch Gas-Stripping abgetrennt und an Aktivkohle adsorbiert wird. Dieses Verfahren erlaubt, die Ethanolkonzentration während der Fermentation in dem Bereich um 6%(w/v) einzustellen.

Für die Ethanolherstellung aus lignocellulosischer Biomasse, die die Fermentation von C5-Zuckern erfordert, ist eine solche Kontrolle bei 6%(w/v) jedoch nicht ausreichend. Beispielsweise konnten Dominguez et al. (Biotech. Bioeng., 2000, Vol. 67, S. 336-343) zeigen, dass die Umsetzung von C5-Zuckern zu Ethanol mit der Hefe *Pichia stipitis* bei nur 2%(w/v) Ethanol inhibiert wird. Dominguez et al. haben daher ein Verfahren entwickelt, bei dem die Ethanolkonzentration während der Fermentation von Xylose unter 2%(w/v) gehalten werden kann, wobei nach erfolgtem in *situ-Stripping* in einem besonders ausgestalteten Fermenter mit Seitenarm das Ethanol an einem eisgekühlten Kondensator kondensiert wird.

US4515892A beschreibt ein Verfahren zur Abtrennung von Ethanol aus Fermentationsbrühen durch Adsorption des Ethanols an einen Adsorber und anschließende Desorption. CN101024846A offenbart gemäß DATABASE WPI Week 200830 (Thomson Scientific) ein Verfahren, in dem hydrophobe Zeolith-Adsorber zur Abtrennung von Ethanol eingesetzt werden, wobei die Desorption mit Kohlendioxid erfolgt.

### Zusammenfassung der Erfindung

Vor diesem Hintergrund bestand die Aufgabe, ein wirtschaftliches Verfahren zur Herstellung von Ethanol durch Fermentierung bereitzustellen, das eine hohe Ethanoiausbeute bei Verwendung von Gemischen aus C5 und C6-Zuckern, wie sie z.B. aus lignocellulosehaltiger Biomasse erhalten werden, erlaubt.

Es wurde überraschenderweise gefunden, dass durch Kombination von *in situ-*Stripping und einem Zeolithadsorber nicht nur die Ethanolkonzentration in der Fermentationslösung über die gesamte Fermentationsdauer bei unter 5%(w/v) gehalten werden kann, sondern zudem die Verwendung des Zeolithadsorbers eine besonders energiesparende Verfahrensführung erlaubt. Es wird somit erfindungsgemäß bereitgestellt ein Verfahren zur Ethanol-Herstellung umfassend:
a. die fermentative Umsetzung von C5- oder C5-und C6-Zuckern zu Ethanol in einer Fermentaiionslösung;
b. die in *situ*-Entfernung des Ethanols durch Gas-Stripping mit Hilfe eines Trägergases, wobei die Ethanol-Konzentration in der Fermentationslösung unter 5%(w/v) gehalten wird;
c. Durchleiten des durch das Gas-Stripping entstandenen Ethanol-Trägergas-Gemisches durch einen Zeolith-Adsorber mit einem SiO₂/Al₂O₃-Verhältnis von 200-1000, wobei Ethanol aus dem Gasgemisch an einen Adsorber adsorbiert wird; und
d. die Desorption des adsorbierten Ethanols von dem Adsorber,
wobei das Trägergas nach Austritt aus dem Adsorber in die Ferementationslösung rückgeführt wird.

### Abbildungen

Abbildung 1 zeigt ein Ausführungsbeispiel des erfindungsgemäßen Verfahrens.
Abbildung 2 zeigt den Verlauf der Ethanolkonzentration während der Fermentation gemäß Beispiel 1.
Abbildung 3 zeigt einen Vergleich zwischen dem erfindungsgemäß verwendeten Zeolith und Aktivkohle bezüglich der Ethanolselektivität.
Abbildung 4 zeigt eine erfindungsgemäße Ausführungsform mit Revolverkonfiguration,

### Detaillierte Beschreibung der Erfindung

### Fermentation von C5- und C6-Zuckern

Für die Fermentation wird eine Lösung mit C5- und C6-Zuckern bereitgestellt. Vorzugsweise enthält die Lösung anfänglich weniger als 200 g/L Zucker, davon weniger als 100 g/L C6-Zucker, besonders bevorzugt weniger als 80 g/L C6-Zucker, ganz besonders bevorzugt weniger als 70 g/L C6-Zucker, und weniger als 100 g/L C5-Zucker, besonders bevorzugt weniger als 35 g/L C5-Zucker, ganz besonders bevorzugt weniger als 30 g/L C5-Zucker. In einer besonders bevorzugten Ausführungsform enthält die Lösung weniger als 120 g/L Zucker, wobei 90 oder mehr % der Zucker C6-Zucker sind. In einer weiteren, besonders bevorzugten Ausführungsform enthält die Lösung weniger als 120 g/L Zucker, wobei 90 oder mehr % der Zucker C5-Zucker sind. In einer weiteren, besonders bevorzugten Ausführungsform enthält die Lösung weniger als 120 g/L Zucker, wobei 20 bis 40% der Zucker C5-Zucker und entsprechend 60 bis 80% C6-Zucker sind. Diese Lösung wird regelmäßig aus kohlenhydrathaltigen Rohsubstraten gewonnen. Es kann notwendig sein, diese Rohsubstrate mittels geeigneter Vorbehandlungsverfahren aufzuschließen und / oder die Kohlenhydrate enzymatisch oder säurekatalysiert zu Zuckermonomeren zu hydrolysieren. Vor der Fermentation kann die Lösung optional aufkonzentriert werden.

Der Begriff "kohlenhydrathaltiges Rohsubstrat" umfasst kohlenhydrathaftige Reinstoffe, Gemische verschiedener Kohlenhydrate sowie komplexe Gemische von Substraten, die Kohlenhydrate enthalten. Kohlehydrathaltiges Material umfasst weiterhin Abfallprodukte aus der Forst- und Landwirtschaft und der Nahrungsmittel verarbeitenden Industrie sowie kommunale Abfälle. Unter die kohlenhydrathaltigen Materialien fällt insbesondere lignocellulosische Biomasse (LCB), welche Cellulose, Hemicellulose und Lignin enthält. LCB aus der Landwirtschaft umfasst unter anderem Getreidestroh und -spelzen (Weizen, Roggen, Gerste, Hafer), Maisstroh und -spindeln, Stallmist, Zuckerrohr-Presskuchen (Bagasse), Zuckerrüben-Pulpe (Zuckerrübenpressschnitzel) und krautige Materialien und Gräser, wie Gertengras, *Sericea Lespedeza,* Rutenhirse (Panicum virgatum, Switchgrass), Elephantengras (Miscanthus, Chinaschilf), und Sudangras (Sorghum sudananse, Sorghum drummondii). LCB in Form von Abfallprodukten aus der Forstwirtschaft umfasst unter anderem Baumrinde, Hackschnitzel und Holzverschnitt. LCB in Form von Rohsubstraten aus der Nahrungsmittelindustrie umfasst unter anderem Fruchtpulpe, Agavenrückstände, Kaffeerückstände und Ölmühlen-Abfälle, wie Rapssamen-Presskuchen und Mühlen-Abwässer. LCB in Form von Rohsubstraten aus der Zellstoff- und Papierindustrie umfasst unter anderem Papierbrei und Papiermühlen-Abwässer. LCB in Form von Rohsubstraten aus kommunalen Abfällen umfasst, ist aber nicht beschränkt auf Papierabfälle, Gemüse- und Fruchtrückstände. Die Fermentationslösung wird bevorzugt aus LCB durch Hydrolyse erhalten. Der Lösung können weitere Zusatzstoffe wie pH-Stellmittel zugegeben werden.

Die bei der Hydrolyse freigesetzten C5-Zucker, gegebenenfalls zusammen mit C6-Zucker, werden durch Fermentation zu Ethanol umgesetzt. Nach der bevorzugten Ausführungsform kommen dabei Hefen oder Bakterien zum Einsatz. Besonders bevorzugt sind C5- und C6-Zucker metabolisierende Hefen und insbesondere solche, deren Fermentationsaktivität bei Ethanolkonzentrationen über 5%(w/v) inhibiert werden.

Nach dem erfindungsgemäßen Verfahren liegt die Temperatur des Fermenters zwischen 10 und 50°C, vorzugsweise zwischen 20 und 40°C.

Die Fermentation erfolgt vorzugsweise im Batch-Betrieb (absatzweise), im fed-batch-Betrieb oder im kontinuierlichen Betrieb. Besonders bevorzugt erfolgt die Fermentation im Batch-Betrieb.

### Das in-situ Stripping

Gemäß der vorliegenden Erfindung erfolgt *in-situ* eine Überführung der flüchtigen Bestandteile, insbesondere des Produkts Ethanol, durch Strippen mit einem inerten Trägergas in die Gasphase. Als Trägergas kommen Gase wie Kohlendioxid, Helium, Wasserstoff, Stickstoff, oder Luft in Frage, sowie Mischungen dieser Gase. Besonders bevorzugt sind Kohlendioxid und Mischungen aus Kohlendioxid und Luft, wodurch bei Bedarf mikroaerobe Bedingungen eingestellt werden können. Ein Vorteil dieser Ausführungsform des erfindungsgemäßen Verfahrens besteht darin, dass das während der Fermentation gebildete Kohlendioxid direkt als Trägergas verwendet werden kann.

Entsprechend des erfindungsgemäßen Verfahrens erfolgt die Fermentation in einem Rührkessel-, oder in einem Schlaufenreaktor oder in einem Air-Lift-Reaktor. Zudem ist der Gasaustausch auch über eine mit dem Fermenter verbundene, externe Gas-Stripping-Säule möglich, die kontinuierlich mit der Fermentationslösung gespeist wird und dessen Auslass wieder zurück in den Fermenter geführt wird. Besonders bevorzugt wird eine solche externe Gas-Stripping-Säule im Gegenstrom und/oder in Kombination mit Füllkörpern für einen erhöhten Stoffaustausch, wie z.B. Raschigringen betrieben.

Die spezifische Begasungsrate liegt vorzugsweise zwischen 0,1 und 10 vvm, besonders bevorzugt zwischen 0,5 und 5 vvm.

Das Stripping wird vorzugsweise bei einem Druck zwischen 0,1 und 2 bar, besonders bevorzugt zwischen 0,5 und 1,1 bar, durchgeführt. Besonders bevorzugt ist ein Stripping bei Unterdruck.

Um ein effizientes Gas-Stripping im Fermenter zu erzielen, werden die Gasblasen vorzugsweise dispergiert. Dies kann mit einem Rührer erfolgen, der so angeordnet ist, dass feine Blasen des Trägergases entstehen.

In einer bevorzugten Ausführungsform erfolgt die *in*-situ-Entfernung von Ethanol aus der Fermentationslösung bei der Fermentationstemperatur. Somit ist keine zusätzliche thermische Energie für die Erwärmung der Fermentationslösung notwendig.

Ein weiterer Vorteil des erfindungsgemäßen Verfahrens besteht darin, dass die durch den Übergang der flüchtigen Substanzen von der Flüssigkeit in die Gasphase abgeführte Verdampfungsenthalpie zur Kühlung des Fermenters beiträgt und somit die erforderliche Kühlleistung zur Konstanthaltung der Temperatur im Fermenter verringert wird.

### Die Adsorption

Gemäß dem erfindungsgemäßen Verfahren wird der den Fermenter verlassende Gasstrom durch eine oder mehrere Säulen geleitet, welche mit einer oder mehreren Arten von Adsorbentien gefüllt sind. Zumindest eine der Säulen enthält Zeolith mit einem SiO₂/Al₂O₃-Verhältnis von 200-1000 als Adsorber. Als weitere Adsorbentien eignen sich Silica, Bentonite, Silicalite, Tone, Hydrotalcite, Aluminiumsilikate, Oxidpulver, Glimmer, Gläser, Aluminate, Clinoptolite, Gismondine, Quartze, Aktivkohlen, Knochenkohle, Montmorilionite, Polystyrole, Polyurethane, Polyacrylamide, Polymethacrylate oder Polyvinylpyridine. In einer besonders bevorzugten Ausführungsform werden ausschließlich Zeolithe als Adsorbentien verwendet.

Verwendet werden Zeolithe, besonders bevorzugt Zeolithe des beta- oder MFI-Typs. Der Zeolith weist ein SiO₂/Al₂O₃-Verhältnis von 200 bis 1000 auf, und besonders bevorzugt ein SiO₂/AlO₃-Verhältnis von 400 bis 800. Besonders bevorzugt sind die synthetischen Zeolithe gemäß US 7,244,409.

Das Massenverhältnis von Adsorbens zu adsorbierten Ethanol liegt vorzugsweise zwischen 1 und 1000, besonders bevorzugt zwischen 5 und 20.

Bei der Adsorption von Ethanol an das Adsorbens (die Adsorbentien) wird die Adsorptionsenthalpie freigesetzt, welche zu einer Erhitzung der Packung führt. Aufgrund der geringen Wärmeleitfähigkeit der beschriebenen möglichen Adsorbensmaterialien und des Hohlraumvolumens innerhalb der Schüttung kann diese Wärme insbesondere bei großen Säulendurchmessern nicht effektiv über die Säulenwand abgeführt werden. In einer bevorzugten Ausführungsform der vorliegenden Erfindung kommen deshalb zusätzlich Heizwendeln innerhalb der Säule zum Einsatz, welche den Austrag der freiwerdenden Adsorptionsenthalpie ermöglichen. Ein Vorteil dieser Ausführungsform besteht darin, dass somit Energie für die nachfolgenden energieerfordernden Prozessschritte gewonnen werden kann.

Entsprechend dem erfindungsgemäßen Verfahren kann über die Heizwendeln innerhalb der Säule die Temperatur beeinflusst und konstant gehalten werden, wodurch die Selektivität des Adsorbens beeinflusst werden kann. In einer bevorzugten Ausführungsform des Verfahrens wird die Selektivität nicht nur durch die Temperatur, sondern auch durch den Druck innerhalb der Säule gesteuert.

Die Temperatur bei der Adsorption des Ethanols liegt vorzugsweise zwischen 10-100°C, besonders bevorzugt zwischen 20 und 50°C. Der Druck liegt vorzugsweise zwischen 0,5-10 bar, besonders bevorzugt zwischen 1 und 2 bar liegt.

Besonders bevorzugt ist es, die Adsorption bei einer Temperatur durchzuführen, die nicht über der Temperatur des Ethanol-Trägergas-Gemisches bei Austritt aus der Fermentationslösung liegt. In einer besonders bevorzugten Ausführungsform werden weder das Ethanol-Gas-Gemisch noch der Adsorber vor der Adsorption erwärmt. Zudem ist es besonders bevorzugt, dass die Adsorption bei Überdruck erfolgt.

Die Menge des verwendeten Adsorbermaterials wird vorzugsweise an die Menge des durch die Fermentation entstehenden Ethanols angepasst. Vorzugsweise beträgt die Menge adsorbierten Ethanols am Ende der Fermentation mindestens 90% der maximalen Ethanolaufnahmemenge des Adsorbers. Sowohl die durch die Fermentation entstehende Ethanolmenge wie auch die maximale Ethanolaufnahmemenge des Adsorbers können vor der Fermentation bestimmt werden. Für die Bestimmung beider Größen erfolgt das Gas-Stripping und die Adsorption genauso wie in Beispiel 2 beschrieben, d.h. eine Lösung mit bekannter Ethanol-Korzentration wird vorgelegt und dann kontinuierlich gestrippt. Während dieser Zeit wird die Ethanol-Konzentration in der Vorlage stündlich gemessen. Wenn sich diese nicht mehr ändert (spätestens nach 24 Stunden) ist die Kapazität des Adsorbermaterials erschöpft. Dann wird der Versuch beendet und das Volumen der Vorlage und die Konzentration des darin erhaltenen Ethanols bestimmt, so dass die Massen von Ethanol und Wasser berechnet werden können. Die Differenzen zwischen den anfangs vorgegebenen Massen und denen nach Versuchsende ergeben die adsorbierten Massen von Ethanol und Wasser (Massenbilanz). Mit Hilfe dieser Werte lassen sich die adsorbierte Ethanol-Konzentration und die Kapazität des Adsorbermaterials bestimmen. Die maximal in der Fermentation gebildete Ethanolmenge kann mit Hilfe der theoretischen Ausbeutekoeffizienten abgeschätzt werden. Die theoretischen Ausbeutekoeffizienten liegen bei 0,51 g Ethanol pro 1 g Glucose beziehungsweise 0,46 g Ethanol pro 1 g Xylose (Lee et al., J. Microbiol. Biotechn., 2001, Vol. 11 (3), S. 384-388). Die in der Praxis erreichten Ethanol-Ausbeuten liegen bei 70-100%, typischerweise bei 90-95% der theoretischen Ausbeuten. Mit Hilfe der zu erwartenden Ethanolmenge und einem Zuschlag von typischerweise 10-20% erfolgt die Berechnung der benötigten Adsorbermenge.

Das Adsorbermaterial kann in einer oder mehreren Säulen enthalten sein. Vorzugsweise werden mehrere, besonders bevorzugt 2 bis 6 Säulen verwendet. Diese Säulen können in Reihe oder parallel geschaltet sein.

Vorteile der Parallelschaltung sind zum einen, dass somit ein quasi-kontinuierlicher Betrieb ermöglicht wird, indem zwei oder mehr Säulen zwischen Ad- und Desorption alternieren, und zum anderen, dass die freiwerdende thermische Energie bei der Adsorption zur Desorption in einer anderen Säule übertragen werden kann, also Adsorption und Desorption zeitgleich an unterschiedlichen Säulen durchgeführt werden können. Die Säulen werden vorzugsweise in einer Revolveranordnung bereitgestellt.

In einer besonders bevorzugten Ausführungsform werden 2 bis 6 Säulen so geschaltet, dass die Säule bzw. Säulen, in denen die Adsorption abläuft, parallel zu der bzw. den Säulen geschaltet wird, in denen die Desorption abläuft. Läuft in mehr als einer Säule die Adsorption ab, so können diese Säulen in Reihe geschaltet werden. So kann bei Verwendung von 6 Säulen z.B. in der "Revolver"-Konfiguration" in den Säulen 1 bis 3 die Adsorption ablaufen, Säule 4 für die Desorption erwärmt werden, in Säule 5 die Desorption ablaufen, und Säule 6 lässt man auskühlen. Die Adsorbersäule wird gewechselt, wenn die Menge adsorbierten Ethanols mindestens 90%, besonders bevorzugt mindestens 95% der maximalen Ethanolaufnahmemenge des Adsorbers in dieser Säule beträgt.

Das erFindungsgemäße Verfahren mit mehreren Adsorptionssäulen eröffnet zudem die Möglichkeit, zwei oder mehr Säulen in Reihe zu schalten, die jeweils mit unterschiedlichen Adsorbentien gefüllt sind, die unterschiedliche Selektivitäten und / oder Kapazitäten aufweisen. Hierbei durchströmt vorzugsweise das Trägergas bei der Adsorption die Adsorber in Reihenfolge steigender Ethanol-Bindeselektivitäten (in Bezug auf Wasser).

Der an Ethanol abgereicherte Gasstrom wird nach Austritt aus der Adsorptionssäule zurück in den Fermenter geführt und steht dann erneut für das Gas-Stripping zur Verfügung.

Die Adsorption kann im Wirbelschichtbetrieb durchgeführt werden.

Mit der erfindungsgemäßen Kombination aus in-situ-Gas-Stripping und Adsorption an Zeolith kann die Ethanolkonzentration in der Fermentationslösung auf unter 5%(w/v), vorzugsweise unter 2%(w/v) über die gesamte Fermentationsdauer gehalten werden. Die Fermentation wird vorzugsweise durchgeführt, solange Ethanol entsteht. Bevorzugte Fermentationsdauern betragen 20 bis 120 Stunden, besonders bevorzugt 30 bis 80 Stunden.

### Die Desorption

Das erfindungsgemäße Verfahren ermöglicht die selektive Desorption des Ethanols vom Adsorbens durch Erhöhung der Temperatur und / oder Verringerung des Drucks innerhalb der Säule. In einer bevorzugten Ausführungsform des Verfahrens wird die thermische Energie über die Säulenwand und ggf. zusätzlich über die Heizwendeln im Inneren der Säule direkt auf die Adsorbenspackung eingebracht. Bevorzugt sind Temperaturen zwischen 25 und 300°C und Absolutdrücke zwischen 0 und 10 bar. Besonders bevorzugt sind Temperaturen zwischen 80 und 180°C, sowie Absolutdrücke bei Unterdruck, vorzugsweise zwischen 0,1 und 1 bar.

Entsprechend des erfindungsgemäßen Verfahrens wird für den Austrag des desorbierten Ethanols aus der Säule ein Trägergas verwendet. Bevorzugt wird hierbei das gleiche inerte Trägergas verwendet, welches auch für das Gas-Stripping eingesetzt wird. In einer Ausführungsform des Verfahrens werden die Temperatur und der Absolutdruck des Trägergases entsprechend den oben beschriebenen Temperaturen und Absolutdrücken innerhalb der Säule eingestellt. Für diesen Zweck eignen sich vorgeschaltene Wärmetauscher und / oder Drosseln beziehungsweise Kompressoren.

Die Desorption kann im Wirbelschichtbetrieb durchgeführt werden.

### Weitere Aufreinigung

Eine bevorzugte Ausführungsform des Verfahrens sieht die Kondensation des desorbierten Ethanol-Gases vor. Nach einer bevorzugten Ausführungsform des Verfahrens wird dazu der Gasstrom komprimiert und / oder gekühlt durch Verwendung von einem oder mehreren Kompressoren und / oder einem oder mehreren Wärmetauschern und / oder einer oder mehreren Kühlfallen. Besonders bevorzugt sind Gegenstromwärmetauscher. In einer weiteren bevorzugten Ausführungsform des Verfahrens werden durch die Reihenschaltung von zwei oder mehreren Wärmetauschern und / oder Kühlfallen mit unterschiedlichen Kühltemperaturen Kondensate mit unterschiedlichen Ethanol-Konzentrationen erhalten. Zudem eröffnet dies die Möglichkeit der selektiven Kondensation noch vorhandener weiterer Begleitstoffe, wie Wasser oder anderen flüchtiger Substanzen.

Bei der Kondensation wird die Kondensationsenthalpie freigesetzt. Gemäß einer bevorzugten Ausführungsform des Verfahrens wird diese thermische Energie mittels Wärmetauschern auf vorausgehende und / oder mögliche nachfolgende, energieerfordernde Verfahrensschritte übertragen. Gemäß einer besonders bevorzugten Ausführungsform des Verfahrens handelt es sich bei diesen energieerfordernden Verfahrensschritten um die vorangegangene Desorption des Ethanols und / oder eine mögliche nachfolgende Rektifikation.

Entsprechend einer weiteren Ausführungsform des Verfahrens wird das erhaltene kondensierte Ethanol weiter aufgereinigt und aufkonzentriert. Ein typischer Begleitstoff des Ethanols im Kondensat ist Wasser. Die Entfernung von Wasser und / oder weiterer Begieitstoffie kann durch Rektifikation erfolgen.

In einer bevorzugten Ausführungsform des Verfahrens wird bei der Kondensation des Ethanols die Temperatur knapp unterhalb des Siedepunkts des entstehenden Kondensats gehalten, so dass die zu rektifizierende Ethanol-Lösung nahe am Siedepunkt an die Rektifikation übergeben wird, wodurch der Energieaufwand für die Rektifikation reduziert wird. Mittels Brüdenkompression kann der Energiebedarf der Rektifikation noch weiter reduziert werden.

Das im Sumpf der Rektifikationskolonne erhaltene Wasser kann in den Fermenter zurückgeführt werden. Am Kopf der Kolonne wird das Azeotrop zwischen Ethanol, und Wasser erhalten. Um wasserfreies Ethanol zu erhalten, ist es möglich nachfolgend geeignete Trennverfahren einzusetzen, wie zum Beispiel die Entfernung von Wasser mittels eines Molekularsiebs oder durch Einsatz selektiver Membranverfahren. Ebenso ist es möglich die Lage des Azeotrops über die Veränderung des Drucks bei der Rektifikation zu verschieben.

Entsprechend einer alternativen Ausführungsform des Verfahrens wird vor der Kondensation des desorbierten Ethanol-Gases dieses über eine weitere Säule zur Entfernung von Begleitstoffen aus der Gasphase geleitet. Bevorzugt ist hierbei die Entfernung von Wasser mittels eines Molokularsiebs. Außerdem ist bei dieser alternativen Ausführungsform die Anwendung von Dampfpermeation möglich.

Eine weitere alternative Ausführungsform des Verfahrens sieht vor, das nach der Desorption erhaltene Kondensat einer Pervaporation zuzuführen, so dass absolutes Ethanol erreicht werden kann.

Gemäß dem Verfahren wird der Trägergasstrom, der durch die Kondensation des Ethanols und anderer möglicher Begleitstoffe rückgewonnen wird, im Kreislauf geführt, so dass für das Gas-Stripping im Fermenter keine oder eine nur geringe externe Zufuhr von Trägergas notwendig ist.

### Besonders bevorzugte Ausführungsformen

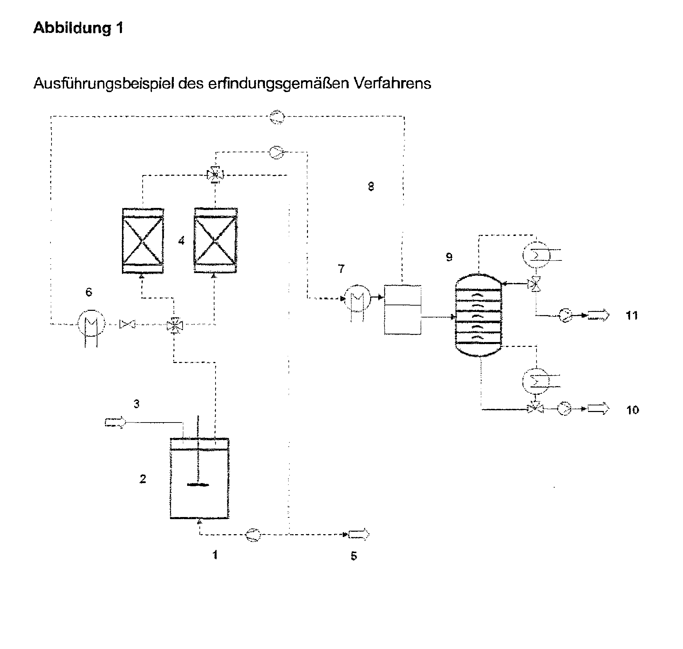

Abbildung 1 zeigt eine mögliche Ausführungsform des erfindungsgemäßen Verfahrens. Ein inerter Trägergasstrom (1) wird zum Gas-Stripping in den Fermenter (2) eingeblasen. Innerhalb des Fermenters wird LCB zu Ethanol, fermentiert, wobei Hilfsstoffe (3) wie pH-Stellmittel zugegeben werden.

Das den Fermenter verlassende Gas, welches Ethanol und andere flüchtige Bestandteile enthält, wird durch eine Adsorptionssäule (4) geleitet, die das Ethanol selektiv adsorbiert. Um einen quasi-kontinuierlichen Betrieb zu gewährleisten, werden zwei oder mehrere Säulen parallel und/oder in Reihe geschaltet. Ein thermischer Austausch zwischen den Säulen wird durch interne Heizwendeln erreicht.

Ein Teil des Trägergasstroms wird aufgrund des fermentativ gebildeten Kohienstoffdioxids abgeführt (5).

Zur Desorption des adsorbierten Ethanols wird die Temperatur und / oder der Druck innerhalb der Säulen (4) verändert. Der für den Austrag des desorbierten Ethanols notwendige Trägergasstrom wird über einen Wärmetauscher (6) und / oder Drosseln entsprechend eingestellt.

Das bei der Desorption die Säule verlassende Gas wird anschließend mittels Kompression und / oder Kühlung kondensiert (7). Der somit regenerierte Trägergasstrom (8) wird rückgeführt.

Das Kondensat wird weiter aufgereinigt und aufkonzentriert, indem dieses einer Rektifikationskolonne (9) zugeführt wird. Am Sumpf der Kolonne wird Wasser erhalten (10), am Kopf der Kolonne das Azeotrop zwischen Ethanol und Wasser (11).

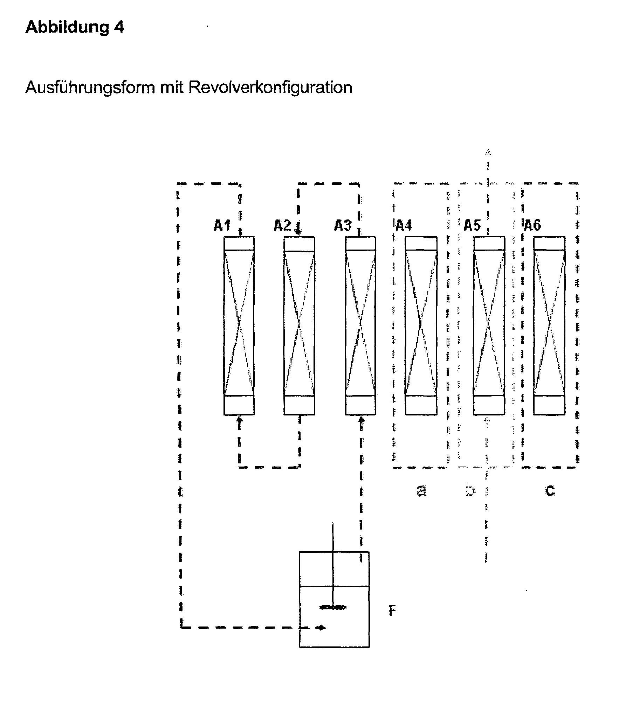

Abbildung 4 zeigt die "Revolverkonfiiguration, bei der drei Säulen (A1-A3) für die Adsorption aus dem vom Fermenter (F) stammenden Stripping-Gas in Reihe geschaltet sind. Die Säulen A4 bis A6 sind parallel geschaltet. Die Säule A4 wird erwärmt (a), die Säule A5 desorbiert (b) und die Säule A6 abgekühlt (c). Nach Ende der Taktzeit gelangt Säule A3 in die Erwärmungsphase (a), A4 wird desorbiert (b) und A5 abgekühlt (c). Für die Adsorption sind dann die Säulen A6, A1 und A2 in Reihe geschaltet. Nach 6 Taktzeiten wird wieder dieselbe Säule desorbiert wie zu Beginn, so dass ein Zyklus vollendet ist und ein neuer beginnt.

Erfindungsgemäß besonders bevorzugt ist folgendes Verfahren:
Verfahren zur Ethanol-Herstellung umfassend:
   a) die fermentative Umsetzung von C5- und C6-Zuckem zu Ethanol in einer Fermentationslösung;
   b) die *in situ*-Entfernung des Ethanols durch Gas-Stripping mit Hilfe eines Trägergases, wobei die Ethanol-Konzentration in der Fermentationslösung unter 5%(w/v) gehalten wird;
   c) Durchleiten des durch das Gas-Stripping entstandenen Ethanol-Trägergas-Gemisches durch einen Adsorber, wobei Ethanol aus dem Gasgemisch an einen Adsorber in einer ersten Säule adsorbiert wird;
   d) die Desorption von adsorbiertem Ethanol von einem Adsorber in einer zweiten Säule,
   e) wobei die durch die Adsorption in der ersten Säule entstehende Wärme zum Aufheizen der zweiten Säule genutzt wird;
   f) wobei das Trägergas nach Austritt aus dem Adsorber in die Fermentationslösung rückgeführt wird; und
   g) Aufkonzentrierung des desorbierten Ethanols.

Der Adsorber ist ein Zeolith mit einem SiO₂/Al₂O₃-Verhältnis von 200 bis 1000. Außerdem beschrieben wird folgendes Verfahren:
Verfahren zur Ethanol-Herstellung umfassend:
   a) die fermentative Umsetzung von C6-Zuckern zu Ethanol in einer Fermentationslösung, welche C5- und C6-Zucker enthält;
   b) die Entfernung des Ethanols aus der Fermentationslösung durch Gas-Stripping mit Hilfe eines Trägergases,
   c) die anschließende fermentative Umsetzung von C5-Zuckem zu Ethanol in der Fermentationslösung, wobei eine *in situ*-Entfernung des Ethanols durch Gas-Stripping mit Hilfe eines Trägergases durchgeführt und die Ethanol-Konzentration in der Fermentationslösung unter 5%(w/v) gehalten wird;
   d) Durchleiten des durch das Gas-Stripping entstandenen Ethanol-Trägergas-Gemisches durch einen Adsorber, wobei Ethanol aus dem Gasgemisch an einen Adsorber adsorbiert und das Trägergas nach Austritt aus dem Adsorber in die Fermentationslösung rückgeführt wird.
Weiterhin beschrieben wird
   folgendes Verfahren:
Verfahren zur Ethanol-Herstellung umfassend:
   a) die fermentative Umsetzung von C6-Zuckem zu Ethanol in einer Fermentationslösung, welche C5- und C6-Zucker enthält, in einem ersten Reaktor;
   b) die anschließende, schrittweise oder kontinuierliche Zuführung der Ethanol- und C5-Zucker-enthaltenden Fermentationslösung in einen zweiten Reaktor;
   c) die fermentative Umsetzung der C5-Zucker in der Fermentationslösung zu Ethanol im zweiten Reaktor;
   d) die *in situ*-Entfernung des Ethanols aus dem zweiten Reaktor durch Gas-Stripping mit Hilfe eines Trägergases;
   e) Durchleiten des durch das Gas-Stripping entstandenen Ethanol-Trägergas-Gemisches durch einen Adsorber, wobei Ethanol aus dem Gasgemisch an einen Adsorber adsorbiert wird;
   f) die Desorption von adsorbiertem Ethanol, wobei das Trägergas nach Austritt aus dem Adsorber in die Fermentationslösung des zweiten Reaktors rückgeführt wird,
   wobei die Zuführung der Ethanol- und C5-Zucker-enthaltenden Fermentationslösung in den zweiten Reaktor derart erfolgt, dass die Ethanolkonzentration in der Fermentationslösung im zweiten Reaktor unter 5%(w/v) gehalten wird.

In diesem Verfahren wird die Ethanol-Konzentration bevorzugt dadurch unter 5%(w/v) gehalten, dass die Zuflussrate der Ethanol- und C5-Zucker-enthaltenden Fermentationslösung in den zweiten Fermenter und / oder die Begasungsrate während des Gas-Strippings und / oder die Adsorbermenge angepasst werden.

Der Adsorber enthält Zeolith. In einer besonders bevorzugten Ausführungsform wird die bei der Adsorption freigesetzte Wärme für die Desorption genutzt. Weiterhin ist es bevorzugt, die Ethanol-Konzentration in der Fermentationslösung unter 5%(w/v) zu halten.

Die Fermentation, das Gas-Stripping, die Ad- und Desorption sowie die Endreinigung sind vorzugsweise thermisch miteinander verknüpft, so dass die Energiekosten zur Aufreinigung des Endproduktes Ethanol gegenüber konventionellen Verfahren deutlich reduziert werden.

### Beispiele

Die Erfindung wird nachstehend anhand nicht-beschränkender Beispiele näher erläutert.

### Beispiel 1- In-situ-Abtrennung von Ethanol während der Fermentation

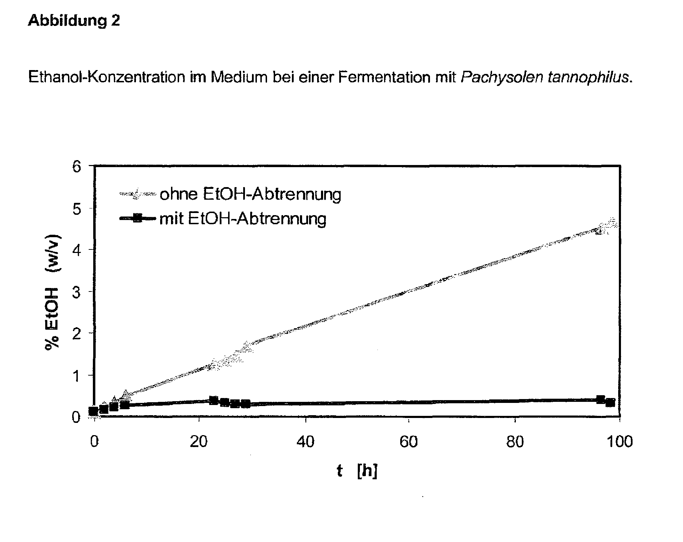

*Pachysolen tannophilus* (DSMZ No. 70352, Braunschweig) wurde mit und ohne in-situ-Abtrennung von Ethanol unter ansonsten identischen Bedingungen für 100 Stunden bei 30°C fermentiert. Das Fermentationsmedium bestand aus 5 g/L Bacto TM Yeast Extract (Becton, Dickinson Co., Frankreich), 6,7 g/L 1x Difco Yeast Nitrogen Base w/o Amino Acids (Becton, Dickinson Co., Frankreich), auf 350 mL H₂O dest. Zwei je 350 mL Kulturen wurden in 1 L Schott-Glasflaschen mit gasdichten GL 45 Flaschen-Mehrfachverteiler (Bola, Grünsfeld), die drei GL14 Zugänge für Schlauchverschraubungen hatten, angesetzt. Als Kohlenstoffquelle wurde jeweils 35g Glucose im Fed-Batch-Betrieb verwendet. Ein Zugang wurde zur Probenabnahme verwendet. Durch einen weiteren Zugang wurde das Trägergas (Stickstoff) über einen PA 12-8x6x1 Schlauch (Riegler, Bad Urach) und eine Glasfritte in das Fermentationsmedium eingeleitet. Über den dritten Zugang wurde das Trägergas in einem Schlauch vom Flaschenkopf in eine 100 mL Glasfrittensäule, die für die *in-Situ-*Abtrennung mit einem Zeolith (Hergestellt nach US Patent No. 7,244,409 B2) gefüllt ist, geleitet. Für das Referenzexperiment war die Glasfrittensäule nicht gefüllt. Der Gaskreislauf wurde durch eine Membranpumpe (KNF, Freiburg), die zwischen die Glasfrittensäule und die Schott-Glasflaschen geschaltet wurde, mit 1,5 L/min betrieben. Je 2,5%(w/v) Glucose (Sigma-Aldrich, München) wurden bei 0, 24, 48 und 72 Stunden zugefüttert. Die Menge an Ethanol im Fermentationsmedium wurde mittels Gas-Chromatographie (Trace GC, Thermo Fisher) bestimmt. Das Ergebnis des Experiments ist in Abbildung 2 dargestellt. Die GC-Bestimmungen der Ethanol-Konzentrationen in den Medien zeigen, dass durch die *in-situ*-Abtrennung die Ethanol-Konzentration des Fermentationsmediums unter 1 %(w/v) Ethanol gehalten werden kann. lnhibierungen durch die Ethanol-Konzentration werden somit vermieden.

Unter ansonsten identischen Bedingungen wie oben beschrieben, wurde weiterhin eine Mischung von C5- und C6-Zuckern mit und ohne in-situ-Abtrennung von Ethanol fermentiert. Dabei wurden 24,5 g Glucose und 10,5 g Xylose als Kohlenstoffquelle im Batch-Betrieb verwendet.

### Beispiel 2- Aufkonzentrierung einer 7,5%(w/v)-Ethanol-Wasser-Lösung durch Gas-Stripping, Adsorption, Desorption und Kondensation

100 mL einer 7,5%(w/v)-Ethanol-Wasser-Lösung wurden für 24 Stunden mit einem Volumenstrom von 0,5 L/min Luft gestrippt. Es wurden eine Membranpumpe (KNF Neuberger, Freiburg, Deutschland), ein Volumenstromregler (Swagelok, Garching, Deutschland) und eine Gaswaschflasche (VWR, Bruchsal, Deutschland) verwendet. Der Gasstrom wurde durch eine Glassäule geleitet (VWR, Bruchsal, Deutschland), welche mit 91 g des Zeolithen (Hergestellt nach US Patent No. 7,244,409 B2) gepackt war. Im Inneren der Säule befanden sich Heizwendeln. Das Gas-Stripping und die Adsorption fanden bei Raumtemperatur statt. Anschließend wurde die Temperatur mit Hilfe der Heizwendein und über die Säulenwand innerhalb von 90 Minuten linear auf 150°C erhöht. Das desorbierte Ethanol wurde in einer Kühlfalle bei - 20°C kondensiert. Der Absolutdruck betrug für Ad- und Desorption jeweils 800 mbar. Der Trägergasstrom wurde im Kreis geführt.

### Ethanol-Konzentrationen:

| | |
|---|---|
| Ausgangslösung: | 7,48%(w/v) |
| Lösung nach Gas-Stripping: | 2,15%(w/v) |
| Kondensat: | 44,92%(w/v) |

### Beispiel 3 - Selektive Adsorption an Aktivkohle und Zeolith

Das Gas-Stripping und die Adsorption erfolgten genauso wie in Beispiel 2 beschrieben. Allerdings wurde das Volumen der Vorlage auf 1 L erhöht, so dass die Konzentrationsänderung durch die Ethanol-Adsorption relativ gering und damit näherungsweise konstant ist ("steady state"). Die adsorbierte Ethanol-Konzentration wurde durch Gas-Chromatographie (Trace GC, Thermo Fisher) bestimmt.

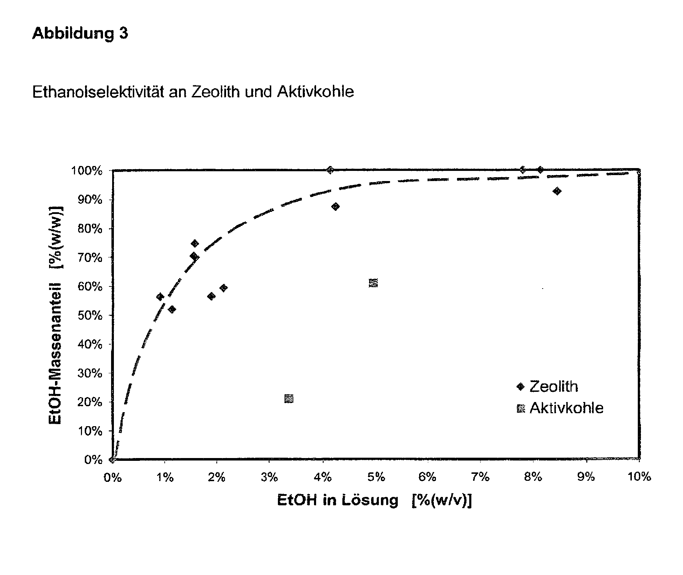

Bei Walsh et al. werden in Table IV, zwei Versuche aufgelistet, bei denen die Ethanol-Konzentration in der Flüssigkeit unter 5%(w/v) lag (4,94%(wlv) und 3,37%(w/v). Berechnet man die Ethanol-Massenanteile aus den adsorbierten Massen, so ergibt sich jeweils 61%(w/w) und 21%(w/w) (siehe Grafik, rote Punkte). Im erfindungsgemäßen Verfahren ergeben sich somit mit dem verwendeten Zeolithen deutlich höhere Ethanol-Massenanteile (siehe Abbildung 3).

## Patentansprüche

1. Verfahren zur Ethanol-Herstellung umfassend:
a. die fermentative Umsetzung von C5- oder C5- und C6-Zuckem zu Ethanol in einer Fermentationslösung;
b. die *in* situ-Entfernung des Ethanols durch Gas-Stripping mit Hilfe eines Trägergases, wobei die Ethanol-Konzentration in der Fermentationslösung unter 5%(w/v) gehalten wird;
c. Durchleiten des durch das Gas-Stripping entstandenen Ethanol-Trägergas-Gemisches durch einen Zeolith-Adsorber, wobei der Zeolith-Adsorber ein SiO₂/Al₂O₃-Verhältnis von 200 bis 1000 aufweist, und wobei Ethanol aus dem Gasgemisch an einen Adsorber adsorbiert wird; und
d. die Desorption des adsorbierten Ethanols von dem Adsorber,
wobei das Trägergas nach Austritt aus dem Adsorber in die Fermentationslösung rückgeführt wird.

2. Verfahren nach Anspruch 1, wobei die Menge adsorbierten Ethanols am Ende der Fermentation mindestens 90% der maximalen Ethanolaufnahmemenge des Adsorbers beträgt.

3. Verfahren nach einem oder mehreren der Ansprüche 1 und 2, wobei die Adsorption bei einer Temperatur erfolgt, die nicht über der Temperatur des Ethanol-Trägergas-Gemisches bei Austritt aus der Fermentationslösung liegt.

4. Verfahren nach einem oder mehreren der vorhergehenden Ansprüche, wobei das Gas-Stripping in einer mit dem Fermenter verbundenen Gas-Stripping-Säule durchgeführt wird, die kontinuierlich mit der Fermentationslösung gespeist wird und dessen Auslass wieder zurück in den Fermenter geführt wird.

5. Verfahren nach Anspruch 4, wobei die Gas-Stripping Säule im Gegenstrom betrieben wird und / oder Füllkörper enthält.

6. Verfahren gemäß einem oder mehreren der Ansprüche 4 und 5 wobei in der Ad-/ Desorptionssäule der Ein- und Austrag von thermischer Energie zusätzlich zur Säulenwand und durch den Trägergasstrom auch über Heizwendeln erfolgt.

7. Verfahren nach einem oder mehreren der vorhergehenden Ansprüche, wobei die spezifische Begasungsrate zwischen 0,1 und 10 vvm, vorzugsweise zwischen 0,5 und 5 vvm liegt.

8. Verfahren nach einem oder mehreren der vorhergehenden Ansprüche, wobei Kohlenstoffdioxid, welches während der Fermentation gebildet wird, als Trägergasstrom eingesetzt wird.

9. Verfahren gemäß einem oder mehreren der vorhergehenden Ansprüche, wobei die Temperatur bei der Adsorption des Ethanols zwischen 10-100°C, vorzugsweise zwischen 20 und 50°C und der Druck zwischen 0,5-10 bar, vorzugsweise zwischen 1 und 2 bar liegt.

10. Verfahren gemäß einem oder mehreren der Ansprüche 4 bis 6, wobei mehrere Ad-/ Desorptionssäulen parallel und / oder in Reihe geschalten werden.

11. Verfahren gemäß Anspruch 10, wobei die Säulen mit unterschiedlichen Arten von Adsorbentien gefüllt sind.

12. Verfahren gemäß Anspruch 10, wobei die Säulen parallel und/oder in Reihe geschaltet werden und die Adsorption in einer Säule zeitgleich mit der Desorption in einer anderen Säule durchgeführt wird.

## Claims

1. Method for ethanol production comprising:
a. fermentative conversion of C5 or C5 and C6 sugars to ethanol in a fermentation solution;
b. in situ removal of the ethanol by gas stripping by means of a carrier gas, the ethanol concentration in the fermentation solution being kept below 5% (w/v);
c. passage of the ethanol/carrier gas mixture produced by gas stripping through a zeolite adsorber, the zeolite adsorber having a SiO₂/Al₂O₃ ratio from 200 to 1000, and ethanol being adsorbed from the gas mixture to an adsorber; and
d. desorption of the adsorbed ethanol from the adsorber,
the carrier gas after leaving the adsorber being returned to the fermentation solution.

2. Method according to claim 1, the quantity of adsorbed ethanol at the end of fermentation being at least 90% of the maximum ethanol adsorption quantity of the adsorber.

3. Method according to one or more of claims 1 and 2, adsorption taking place at a temperature which is not above the temperature of the ethanol/carrier gas mixture on leaving the fermentation solution.

4. Method according to one or more of the preceding claims, gas stripping being carried out in a gas stripping column which is connected to the fermenter and which is continuously supplied with the fermentation solution and of which the outlet leads back to the fermenter again.

5. Method according to claim 4, the gas stripping column being operated in countercurrent and/or containing packing material.

6. Method according to one or more of claims 4 and 5, the input and output of thermal energy in the adsorption/desorption column taking place, in addition to the column wall and through the carrier gas stream, over heating coils as well.

7. Method according to one or more of the preceding claims, the specific gassing rate being between 0.1 and 10 vvm, preferably between 0.5 and 5 vvm.

8. Method according to one or more of the preceding claims, carbon dioxide which is formed during fermentation being used as the carrier gas stream.

9. Method according to one or more of the preceding claims, the temperature during adsorption of the ethanol being between 10 and 100°C, preferably between 10 and 50°C, and the pressure being between 0.5 and 10 bars, preferably between 1 and 2 bars.

10. Method according to one or more of claims 4 to 6, several adsorption/desorption columns being connected in parallel and/or in series.

11. Method according to claim 10, the columns being filled with different types of adsorbents.

12. Method according to claim 10, the columns being connected in parallel and/or in series and adsorption being carried out in one column at the same time as desorption in another column.

## Revendications

1. Procédé de production d'éthanol, comprenant :
a. la conversion par fermentation de sucres C5 ou C5 et C6 en éthanol dans une solution de fermentation ;
b. l'extraction *in situ* de l'éthanol par entraînement gazeux au moyen d'un gaz porteur, la concentration d'éthanol dans la solution de fermentation étant maintenue sous 5 % (p/v) ;
c. la traversée d'un adsorbeur à zéolite par le mélange gaz porteur - éthanol obtenu par entraînement gazeux, l'adsorbeur à zéolite présentant un rapport SiO₂/Al₂O₃ de 200 à 1000, et l'éthanol du mélange gazeux étant adsorbé sur un adsorbeur ; et
d. la désorption de l'éthanol adsorbé par l'adsorbeur, le gaz porteur étant reconduit vers la solution de fermentation après sortie de l'adsorbeur.

2. Procédé selon la revendication 1, où la quantité d'éthanol absorbé à la fin de la fermentation représente au moins 90 % de la quantité d'adsorption d'éthanol maximale de l'adsorbeur.

3. Procédé selon l'une ou plusieurs des revendications 1 et 2, où l'adsorption a lieu à une température qui ne dépasse pas la température du mélange gaz porteur - éthanol à la sortie de la solution de fermentation.

4. Procédé selon l'une ou plusieurs des revendications précédentes, où l'entraînement gazeux est effectué dans une colonne d'entraînement gazeux reliée au bioréacteur, laquelle est alimentée en solution de fermentation de manière continue et dont l'évacuation est reconduite vers le bioréacteur.

5. Procédé selon la revendication 4, où la colonne d'entraînement gazeux est mise en service à contre-courant et/ou contient des corps de remplissage.

6. Procédé selon l'une ou plusieurs des revendications 4 et 5, où dans la colonne d'adsorption/désorption, l'apport et l'évacuation d'énergie thermique sont également effectués au moyen d'enroulements chauffants, en complément de la paroi de colonne et du courant de gaz porteur.

7. Procédé selon l'une ou plusieurs des revendications précédentes, où le débit d'aération spécifique est compris entre 0,1 et 10 vvm, de préférence entre 0,5 et 5 vvm.

8. Procédé selon l'une ou plusieurs des revendications précédentes, où le dioxyde de carbone formé pendant la fermentation est utilisé comme courant de gaz porteur.

9. Procédé selon l'une ou plusieurs des revendications précédentes, où la température lors de l'adsorption de l'éthanol est comprise entre 10 et 100 °C, de préférence entre 20 et 50 °C, et la pression entre 0,5 et 10 bar, de préférence entre 1 et 2 bar.

10. Procédé selon l'une ou plusieurs des revendications 4 à 6, où plusieurs colonnes d'adsorption/désorption sont disposées en parallèle et/ou en ligne.

11. Procédé selon la revendication 10, où les colonnes sont remplies d'adsorbants de différents types.

12. Procédé selon la revendication 10, où les colonnes sont disposées en parallèle et/ou en ligne et où l'adsorption dans une colonne est effectuée simultanément à la désorption dans une autre colonne.
